# EUROPEAN PATENT APPLICATION

(11) **EP 3 922 185 A1**
(43) Date of publication of application: **15.12.2021**
(21) Application number: 20179497.1
(22) Date of filing: 11.06.2020
(51) Int. Cl.: A61B 6/12, A61B 6/00, A61B 90/00

(54) **DETERMINING OF PATIENT MOVEMENT FOR MEDICAL IMAGING**

(71) Applicant: Koninklijke Philips N.V., 5656 AG Eindhoven (NL)
(72) Inventor: WEISS, Steffen, 5656 AE Eindhoven (NL); VOGTMEIER, Gereon, 5656 AE Eindhoven (NL); JOHNSON, Mark, 5656 AE Eindhoven (NL); LEUSSLER, Christoph, 5656 AE Eindhoven (NL)
(74) Representative: Philips Intellectual Property & Standards

(57) **Abstract**

This disclosure provides a system (100) for determining patient (P) movement for a medical imaging system, comprising at least one marker (110), and at least one data processing unit (120), wherein the marker (110) is a solid configured to be swallowed by the patient (P). The marker (110) comprises a landmark forming component (111) configured to be detectable within the patient during a medical imaging procedure to determine the movement of the patient. The at least one data processing unit (120) is configured to obtain patient information data and/or medical imaging information data at least indicative for a type of medical imaging procedure intended for the patient. The at least one data processing unit (120) utilizes a computational model to estimate, based on one or more of the patient information data, the medical imaging information data and a configuration of the at least one marker, a position and/or distribution of the at least one marker inside the patient to a certain time and/or over a period of time after swallowing by the patient (P). The data processing unit (120) is configured to generate, based on the estimation of a distribution of the at least one marker, control data for timely controlling the medical imaging procedure.

## Description

### FIELD OF THE INVENTION

The present invention relates to medical imaging. In particular, the present invention relates to a system for determining patient movement for a medical imaging device or system, a marker for a system for determining patient movement for a medical imaging system, the use of such a marker for determining patient movement in medical imaging, and a computer-implemented method of determining patient movement for medical imaging.

### BACKGROUND OF THE INVENTION

In medical imaging, it may be crucial to determine patient movement during a medical imaging scan or the like. For example, the patient movement to be determined may comprise determining a respiratory state of a patient, a breathing phase of a patient, or the like. Such determination may be used to trigger or gate medical imaging.

Recently, different approaches for determining such a patient movement have been proposed. For example, a sensor arrangement may be used, which requires correct placement of the sensor relative to the patient. Further, for example, an imaging-data-based sensing approach may be used, wherein the computational efforts for determining the patient movement using imaging analysis is high. Further, particularly automatic detection of the patient movement may be difficult to be determined from medical imaging data, since the position of the relevant body part of the patient may be difficult to determine.

### SUMMARY OF THE INVENTION

There may, therefore, be a need to further improve determining patient movement for medical imaging. The object of the present invention is solved by the subject-matter of the independent claims, wherein further embodiments are incorporated in the dependent claims.

According to a first aspect, there is provided a system for determining patient movement for a medical imaging device or system. The device or system comprises:
- at least one marker, and
- at least one data processing unit,
- wherein the marker is a solid configured to be swallowed by the patient, wherein the marker comprises a landmark forming component configured to be detectable within the patient during a medical imaging procedure to determine the movement of the patient,
- wherein the at least one data processing unit is configured to obtain patient information data and/or medical imaging information data at least indicative for a type of medical imaging procedure intended for the patient,
- wherein the at least one data processing unit utilizes a computational model to estimate, based on one or more of the patient information data, the medical imaging information data and a configuration of the at least one marker, an at least approximate position and/or distribution, such as a position of a specific marker in a specific time, of the at least one marker inside the patient to a certain time and/or over a period of time after swallowing by the patient, and
- wherein the at least one data processing unit is configured to generate, based on the estimation of a distribution of the at least one marker, control data for timely controlling the medical imaging procedure.

In this way, determining patient movement for and particularly during medical imaging may be improved. In particular, there is no need for an external sensor to be integrated in a medical imaging environment, since the swallowed marker is detectable, e.g. it can be identified or distinguished from surrounding tissue, in images acquired by the medical imaging system. Further, an image-data-based sensing approach may be improved, since the marker forms a landmark within the body of the patient, allowing for precise determination of patient movement, particularly movement of the patient's respiratory system or the patient's organs involved. In this way, the computational effort for image analysis may be reduced. Further, the patient may be prepared for the examination procedure using the medical imaging device or system by swallowing one or more markers over time, where the marker enters, for example, the digestive tract and serve as an internal landmark. Preferably, the marker is configured to be automatically segmented on raw 3DRX and CT images and MR images, by, for example, having a distinct shape and/or a distinct material, or the like. Further, the system may be applied to a MR Linac therapy system, wherein the determined movement, e.g. the movement of the patient's respiratory system, is used to control the MR Linac therapy system. For example, the detection of the marker by the imaging device may also be used to control an MR LINAC radiation beam or an MR-PET imaging system, or a therapy device thereof. This control may comprise a start-stop control, a change of radiation intensity, an adaptation of a therapy plan, or the like. Further preferably, the marker position and/or distribution within the patient's body over time may be used to derive the patient movement, such as a respiratory state and/or breathing phase of the patient. For example, the motion of e.g. the diaphragm, the bowel or the like may be derived or determined, utilizing the at least one marker. Preferably, the determined patient movement, e.g. the respiratory state and/or breathing phase, may be used to trigger or gate the medical imaging examination procedure.

The at least one data processing unit may comprise one or more of a processor, a memory, a data interface to receive data from and or provide data to the system for determining patient movement and/or the medical imaging device or system.

As used herein, the computational model may be broadly understood as a mathematical model carried out on a computer and configured to predict and/or estimate the behavior of or the outcome of a real-world or physical system, which is here, for example, the digestive system of a human, e.g. a patient. The computational model may realize by running one or more computer programs. Further, it may utilize e.g. a computer simulation, or the like, where, for example, the digestive system of a human, preferably with regard to markers, is reproduced and/or modelled, possibly modelled by experiments, etc. The computational model may utilize, for example, an artificial neural network, or the like. In general, the computational model may be adapted to determine, for example, a correlation between swallowing of the marker by the patient, the movement of the marker through the patient and/or an at least approximate position and/or distribution of the marker within the patient at a given time and/or a time window. The output of the computational model may comprise, for example, an estimated and/or predicted position of a certain marker at a certain time, a recommended administration time for swallowing a specific marker, a recommended starting time for the imaging procedure, a scan schedule, a scan protocol, etc.

The at least one marker may have a distinct shape, such as a sphere, rod with spherical end caps, or the like as long as the shape is suitable for detection and/or distinctness in view of the patient's anatomy, to be visible and/or detectable on images acquired by using a Magnetic Resonance image (MRI) imaging device, a Computer Tomography (CT) imaging device, and/or a 3D-rotational X-ray (3DRX) imaging device. In other words, the marker may have a unique geometry to facilitate detection. In particular, the at least one marker may be detectable on raw 3DRX, CT images, and MR images, preferably by fully automatic image processing. For example, automatic image processing may comprise one or more image analysis techniques, such as e.g. 2D and 3D object recognition, feature recognition, image segmentation, motion detection, e.g. single particle tracking, video tracking, optical flow, etc. Particularly due to the distinct shape, the position and/or orientation of the marker can be determined. Further, the marker may have several parts, and may comprise an outer structure and an inner structure. For example, the outer structure may be formed so as to facilitate swallowing of the marker by the patient, while the inner structure may be formed so as to provide an unique geometry. It is noted that the inner structure may, in at least some embodiments, also be referred to as the landmark forming component. Thereby, the outer structure and the inner structure may be formed from different materials. For example, the marker may comprise a material configured to change its properties and to change the marker's shape in response to a pH value to which the marker is exposed, for example in the patient's stomach. In this way, the marker may be configured to e.g. increase its size at a desired position within the patient's body. It may further be dissolvable, for example by a suitable pH value. Particularly the outer structure may be dissolvable, e.g. due to a suitable pH value after swallowing, so as to release the inner structure after swallowing.

The patient information data may comprise one or more of a patient profile, comprising e.g. an age of the patient, anatomical data associated with the specific patient, such as e.g. body dimensions, weight of the patient, a health state of the patient, and an actual biomedical and/or a physiological state of the patient.

The medical imaging information data may at least be indicative for a type of medical imaging procedure intended for the patient. Further, the medical imaging information data may comprise one or more of a scheduled or planned time for the medical imaging procedure, a scan protocol, etc.

The control data for timely controlling the medical imaging procedure may comprise, for example, a starting time of the medical imaging procedure, a scan sequence, a scan protocol, or the like.

From a functional perspective, the above system may predict and/or estimate a suitable administration time for one or more markers and the time, when a diagnostic scan is likely to be successful. This means that the above system may give instructions on when the one or more markers should be swallowed by or administered to the patient. Further, based on information about the health state of the patient and/or his actual biomedical and physiological state, or a marker may be selected. A dedicated patient specific marker, that may depend on size and/or weight of the patient, effectiveness, a scan protocol, a type of diagnostic scan, etc., may be selected by the above system. The data processing unit may be connected to a display device on-site at the medical imaging system, or to a mobile computer application, such as a computer app or smartphone app, that may be executed on a computer or a portable computer device, such as a terminal, a mobile, a smartphone, a tablet computer, or the like. The display device may display an indicator for the marker state in the body, e.g. it may show a position of the marker at time. In this way it makes the marker visible to a medical professional, which may also be a remote operator, the patient, etc. Further, the data may also be directly transmitted to the medical imaging system or device and/or a radiation therapy system or device, such as an MR Linac therapy system or device, which may detect the exact time to start the imaging procedure, an imaging sequence, or the like, on basis of the e.g. control data. The control data may comprise, for example, one or more time stamps assigned to the administration time where the marker is to be swallowed, an imaging procedure starting time, an imaging schedule, a scan sequence, a scan protocol, etc. The one or more time stamps may also be used for a scanner setup and a marker (re)administration. The time stamp may also simultaneously be registered by a hospital computer system to record the whole imaging procedure. Not that in at least some embodiments, when marker arrives at the target location, an additional contrast medium or agent may be administered, such as a PET tracer or gadolinium based contrast agent, in order to further improve detection.

The scheduling of the medical imaging procedure may be based, for example, on size, weight and/or age of patient and will be done with the help of a training and guiding tool for the patient. Basic information for the exact scheduling may be the planned time for the imaging procedure and the anatomical data based on the patient's profile. Also at this point, any details of time of passage through the patient's body from previous scans of the same patient will have a very high factor for deciding the best time to swallow the markers. The obtained information may be processed to predict and/or estimate the best time, time sequence etc. for swallowing of the one or more markers to get an optimized local position and/or distribution of the one or more markers inside the body for best resolution of the motion estimation or motion determination.

According to an embodiment, the control data may comprise at least an administration time stamp which is derived from a prediction or estimation of an administration time at which the at least one marker is to be swallowed by the patient.

Thus, the medical imaging procedure may be controlled based on the control data, particularly in an at least semi-autonomous manner.

In an embodiment, the control data may comprise at least a medical imaging starting time stamp which is derived from a prediction or estimation of a medical imaging starting time being dependent from an administration time at which the at least one marker is to be swallowed by the patient.

Thus, the medical imaging procedure may be controlled based on the control data, particularly in an at least semi-autonomous manner.

In another embodiment, the control data may comprise a further time stamp associated with a starting or administration time for administering a contrast medium or agent to the patient. The time stamp may be derived from a prediction and/or estimation, In this way, the contrast medium can be administered at such an appropriate time that it can spread throughout the patient's body according to the estimate or prediction and improve detection.

According to an embodiment, the control data may comprise or feed a medical imaging scan protocol configured to control image acquisition during the medical imaging procedure.

Thus, the medical imaging procedure may be controlled based on the control data, particularly in an at least semi-autonomous manner.

In an embodiment, the at least one marker has a distinct shape, preferably the shape having a spherical portion.

Thus, the marker may be easily detectable on images derived from means of medical imaging image acquisition, since the marker is clearly distinguishable from surrounding tissue. Thereby, due to the distinct shape, the marker's position and/or orientation may be detectable.

According to an embodiment, the at least one marker may have an imbalance, preferably an uneven weight distribution.

In an embodiment, the at least one marker comprises a magnet field reacting component to perform a change of position and/or orientation in response to a magnetic field acting thereon. For example, the landmark forming component may be a magnetic material, which can therefore be influenced by an externally applied magnetic field. From a functional perspective, the landmark forming component may be moved and/or oriented relative to a patient table, a patient's organ, etc. by the applied magnet field.

According to an embodiment, the at least one marker may have first dimensions or a first size prior to swallowing by the patient and may have second dimensions or a second size within the patient by reaction with the body or by a self-triggering mechanism, the second dimensions being greater than the first dimensions to facilitate swallowing of the marker.

This makes it more comfortable for the patient to swallow the marker as it has the smaller first size before and during ingestion, and the larger second size only during and/or after it is swallowed. This makes it easier to swallow the marker as it has the smaller first size before and during ingestion, and the larger second size only during and/or after it is swallowed.

In a further embodiment, the marker may comprise a composition or surface treatment, which may contain or give a taste to the patient. Further, an outer surface of the marker may also be covered by a liquid/cream to facilitate swallowing.

In an embodiment, the landmark forming component may be configured by a solid material responsive to the medical imaging technique used in the medical imaging procedure.

Thus, the marker may be clearly detectable in images acquired by the medical imaging system.

In an embodiment, the material responsive to the medical imaging technique may comprise iron.

For example, the marker may have a, preferably small, iron content, so that it produces a signal void in a magnitude image and the typical pattern according to its magnetic moment in a phase image. The iron content may be chosen such that the size of the signal void does not obscure anatomy. Further, several markers with different iron content may provide that can be chosen depending on the type of medical imaging sequence to be applied. Iron doping may provide distinct patterns as for example two dot-like artefacts at the ends of a rod-shaped tablet.

According to an embodiment, the landmark forming component may comprise a radiation absorbing material.

For example, the marker may be configured having an inner structure, e.g. a core, made from a radiation absorbing material, such as e.g. iodine, calcium, or the like, and a more radiation transparent outer structure, e.g. a shell, that may have, for example, an outer shape that facilitates swallowing. An absorber content may be chosen to have a high absorption but is still not beam stopping to minimize signal artefacts on 3d reconstructions. The shape of the absorbing core may be chosen so that it is clearly detectable on a raw image by means of semi or fully automatic image processing.

In an embodiment, the at least one marker may have a tablet shape in which the landmark forming component is integrated.

Thus, swallowing the marker is facilitated for the patient.

According to a second aspect, there is provided a marker for a system for determining patient movement for a medical imaging system. This particularly applies to the system according to the first aspect. The marker comprises:
- an outer structure,
- a landmark forming component,
- wherein the marker is a solid, the outer structure being configured to facilitate swallowing by the patient,
- wherein the landmark forming component is configured to be detectable within the patient during a medical imaging procedure to determine the movement of the patient.

For example, the outer structure, e.g. a shell, may be covered by e.g. a friction-reducing treatment, such as providing a, preferably thin, film. Optionally, the friction-reducing treatment may have a comfortable taste. Further, the surface of the marker may be covered by a liquid/cream to optimize swallowing.

Generally, the design of the marker intended for 3DRX and/or CT may differ from that of the marker intended for MR. For example, a 3DRX and/or CT marker may be designed with an inner structure, e.g. a core, made from a radiation absorbing material, such as iodine, calcium, or the like, and a more radiation transparent outer structure, e.g. a shell, that may have an outer shape to facilitate swallowing. An absorber content may be chosen to have high absorption but still not beam stopping to minimize signal artefacts on 3D reconstructions. The shape of the absorbing core may be chosen so that it is clearly detectable on the raw images by fully automatic image processing. An MR marker may be designed as, for example, a tablet. It may comprise some, preferably small, iron content, so that it may produce a signal void in a magnitude image and/or a typical pattern according to its magnetic moment in a phase image. The iron content may be chosen such that the size of the signal void does not obscure the patient's anatomy. In a least some embodiments, several markers with different iron content may be provided, adapted to be chosen depending on the e.g. a type of MR sequence to be applied. Optionally, iron doping may provide distinct patterns as, for example, two dot-like artefacts at the ends of a rod-shaped tablet. Because scan resolution for fast MR imaging is equal or less than 5mm, a marker tablet may contain a material that is adapted to be compact during swallowing and are designed as tablets with some defined small iron content so that they produce a signal void in the magnitude image and the typical pattern according to their magnetic moment in phase image. The iron content is chosen such that the size of the signal void does not obscure anatomy. Hence, several markers with different iron content are provided that can be chosen depending on the type of MR sequence to be applied. Iron doping may provide distinct patterns as for example two dot-like artefacts at the ends of a rod-shaped tablet. Because scan resolution for fast MR imaging is equal or less than 5mm, tablets may contain a material that is compact during swallowing and swells or extends once digested. For example, this may be induced by a chemical or biochemical reaction, where CO₂ may be released, inflating the marker or a part of it. Thus, the outer diameter may be increased, at least for a period of time depending on the duration of gas confinement. This will enable the distinct patterns mentioned above to cover larger distances and thus be identifiable at low scan resolution and to swell or extends during digestion.

This allows enabling the distinct patterns mentioned above to cover larger distances and thus be detectable even at low scan resolution.

According to an embodiment, the marker may be formed with a, preferably flexible, housing, which may be adapted to accommodate a set of individual markers. The individual markers may be released within the patient's body, and may, optionally, be adapted to different absorbing locations in the body. Optionally, the individual markers may be released simultaneously, so that a clustering of markers can be applied. Alternatively or additionally, the individual markers may be released in a defined way. Individual markers may contain different concentration of contrast agent, or the like.

In an embodiment, the marker may comprise a, preferably dissolvable, actuator device, which can hold the marker inside the body at a certain position for a certain time interval. For this purpose, the actuator may be adapted to comprise an abutment means and/or friction enhancing means, such as a spring-like means, spring means, etc. Optionally, the abutment means and/or friction enhancing means may be adapted to be dissolved in and/or absorbed by the human body after a certain time interval.

According to an embodiment, the marker may be formed as a microelectronic marker, which may comprise, for example, a micro transmitter. It may be adapted to modulate a signal depending on a breathing motion of the patient. The signal may be modulated in amplitude and/or phase. The modulated signal may be detectable by an MRI system or a separate electronic receiver located close to a diagnostic scanner. Optionally, if a CT or X-Ray system is used, it may comprise an additional electronic receive infrastructure, which may be located close to the X-Ray source or opposite close to a detector.

In an embodiment, the marker may be a, preferably dedicated, micro MRI marker, which may comprise an active MRI fiducial device. The fiducial device may be adapted to electronically modulate a resonance frequency to a higher or lower frequency. Thus, a contrast may be created by a B1 field enhancement or field cancellation, also referred to as destructive interference. The MRI system may be configured to detect the slight variations in intensity and phase by a separate correlator computer program or software in k-space.

A third aspect relates to the use of a marker according to the second aspect for determining patient movement in medical imaging, particularly for determining a respirational state of the patient during medical imaging. The use comprises:
- administrating the marker to the patient by swallowing it at an estimated and/or predicted administration time,
- starting a medical imaging procedure at an estimated and/or predicted medical imaging starting time, the medical imaging starting time being dependent from the administration time,
- detecting, by detecting means, the marker within the patient, the marker forming a landmark in at least one position within the patient,
- determining, by a data processing unit, based on the landmark formed by the at least one marker and a surrounding anatomy of the patient a respirational state of the patient during the medical imaging procedure, and
- controlling, preferably by the above or another data processing unit, triggering or gating image acquisition during the medical imaging procedure based on the determined respirational state of the patient.

Thus, the respirational state of the patient may be determined accurately.

For example, the detecting means may be a medical imaging device, and particularly a medical imaging scanner or detector. Alternatively or additionally, the detecting means may be a signal receiver, if the marker comprises a transmitter or the like.

According to a fourth aspect, there is provided a computer-implemented method of determining patient movement for medical imaging, comprising:
- obtaining, by a data processing unit, patient information data,
- obtaining, by the data processing unit, medical imaging information data, at least indicative for a type of medical imaging procedure intended for the patient,
- providing, based on at least the patient information data and the medical imaging information data, at least one marker, the marker being a solid configured to be swallowed by the patient, and configured to form a landmark inside the patient detectable over a period of time during the medical imaging procedure to determine the movement of the patient,
- estimating and/or predicting, by the data processing unit, based on at least the patient information data and the medical imaging information data, a position and/or distribution of the marker inside the patient over time after swallowing by the patient, and
- generating, by the data processing unit, based on the estimation of distribution of the marker, control data for timely controlling the medical imaging procedure.

Thus, the medical imaging procedure can be controlled with a high accuracy. According to another aspect, there is provided a computer program element controlling one or more of the devices and systems as described above which, if the computer program element is executed by a data processing unit, is adapted to perform one or more of the methods as described above.

According to another aspect, there is provided a computer-readable medium having stored computer element as described above.

The computer program element can, for example, be a software program but can also be a FPGA, a PLD or any other appropriate digital means.

It should be noted that the above embodiments may be combined with each other irrespective of the aspect involved. Accordingly, the method and/or use may be combined with structural features and, likewise, the system may be combined with features described above with regard to the method and/or use. Advantageously, the benefits provided by any of the above aspects equally apply to all of the other aspects and vice versa.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig 1 shows in a schematic overview a system for determining movement of a patient for a medical imaging system according to an embodiment.
Fig. 2 shows in a block diagram an architecture of a part of a system for determining movement of a patient according to an embodiment.
Fig. 3A shows a marker of a system for determining movement of a patient according to an embodiment.
Fig. 3B shows a marker of a system for determining movement of a patient according to an embodiment.
Fig. 3C shows a marker of a system for determining movement of a patient according to an embodiment.
Fig. 3D shows a marker of a system for determining movement of a patient according to an embodiment.
Fig. 4 shows in flow chart a use of a marker according to an embodiment.
Fig. 5 shows in flow chart a method for determining movement of a patient according to an embodiment.

### DETAILED DESCRIPTION OF EMBODIMENTS

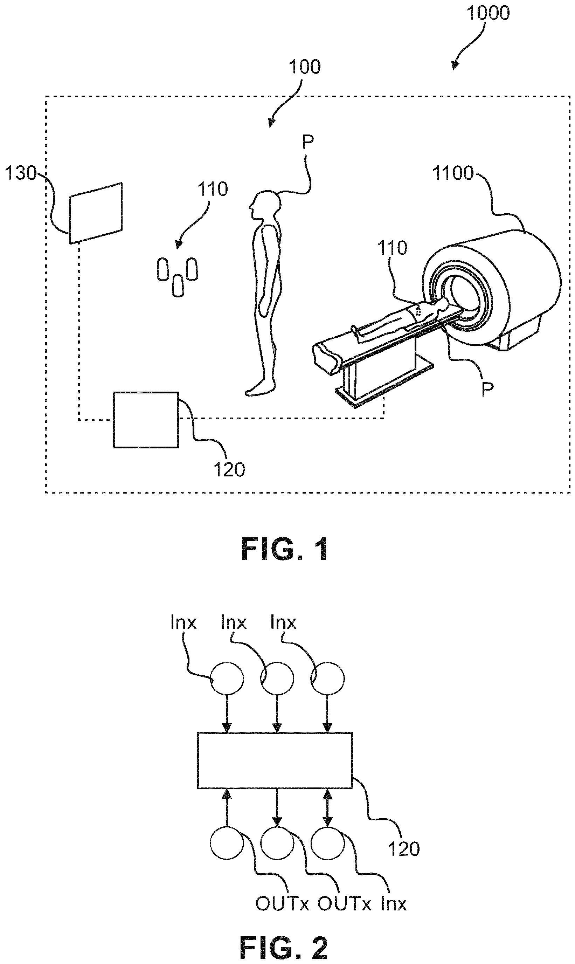

Fig. 1 shows in a schematic overview a system 100 that is configured for determining movement of a patient P for a medical imaging system 1000. The medical imaging system 1000 comprises at least a medical imaging device 1100, which is here, by way of example, represented as a Magnetic Resonance image (MRI) imaging device. It is noted that the medical imaging device 1100 may also be provided as a Computer Tomography (CT) imaging device, and/or a 3D-rotational X-ray (3DRX) imaging device.

The system 100 may be used in preparation of and during operation of the medical imaging system 1000, in particularly during operation of the medical imaging device 1100. It can be integrated into it or provided separately.

The system 100 comprises at least one marker 110, and at least one data processing unit 120.

The marker 110 or the plurality of markers 110 is a solid configured to be swallowed by the patient P, wherein the marker 110 comprises a landmark forming component that is configured to be detectable within the patient during a medical imaging procedure to determine the movement of the patient P. To be detectable within medical imaging data, the marker 110 has, for example, a distinct shape, such as a sphere, a rod with spherical end caps, or the like as long as the shape is suitable for identification and/or distinctness in view of the patient's P anatomy. Thus, it may be detectable on, for example, raw 3DRX, CT images, and/or MR images. The detection is preferably based on a computational image analysis, which can be understood, for example, as the extraction of meaningful information from images, mainly from digital images by means of digital image processing and/or analysis techniques.

The data processing unit 120 is, for example, a suitable computer means, which may comprise one or more of a processor, a memory, one or more data interfaces, one or more communication interfaces, etc. It is configured to obtain patient information data and/or medical imaging information data at least indicative for a type of medical imaging procedure intended for the patient P, wherein the type may comprise the imaging technique itself, i.e. MRI, 3DRX, CT, etc., intended sequences, etc. Further, the data processing unit 120 utilizes a computational model, such as simulation model, to estimate and/or predict, based on one or more of the patient information data, the medical imaging information data and/or a configuration or design of the marker 110, a position and/or distribution of the at least one marker 110 inside the patient P related to time, a certain time, and particularly over a period of time, after swallowing by the patient P. Further, the data processing unit 120 is configured to generate, based on the estimation and/or prediction of a position and/or distribution of the marker 110, control data for timely controlling the medical imaging procedure. These control data may be provided to the system 100 and/or the system 1000. In at least some embodiments, the control data comprises at least an administration time stamp which is derived from an estimation and/or prediction of an administration time at which the marker 110 is to be swallowed by the patient Further, the control data may comprise at least a medical imaging starting time stamp which is derived from an estimation of a medical imaging starting time that is dependent from an administration time at which the marker 110 is to be swallowed by the patient P. Further, in at least some embodiments, the control data comprises or feeds a medical imaging scan protocol configured to control image acquisition during the medical imaging procedure.

Although only one data processing unit 120 is shown here, two or more may be provided, in particular the medical imaging system 1000 and/or the medical imaging device 1100 may comprise its own data processing unit or control system.

As indicated in Fig. 1, the data processing unit 120 is connected to a display device 130. This display device 130 may be configured to display an indicator for the marker state in the body of the patient P. It may be arranged to be visible to the patient P and/or an operator, which may be a remote operator. Additionally or alternatively, the display device 130 may be a display of a portable communication device, such as a smartphone, a home computer, a tablet computer, or the like. Further alternatively, the data may be provided to the medical imaging system 1000 and/or the medical imaging device 1100, which may be start the imaging procedure dependent on the marker state.

Fig. 2 shows in block diagram an architecture of the system 100. The data processing unit 120 is configured to obtain or receive different input parameters, such as on one or more of the patient information data, the medical imaging information data and/or a configuration or design of the marker 110. For example, the data processing unit 120 obtains or receives input data designated by INx and provides output data OUTx. The input data INx may comprise the patient information, a scan info, i.e. information about the medical imaging procedure and/or method, a biomedical and/or physiological state of the patient P, a time stamp related to the planned medical imaging procedure, and/or a scan protocol. The output data OUTx may comprise a marker selection indicating which marker 110 configuration is to be used, and/or a scan protocol.

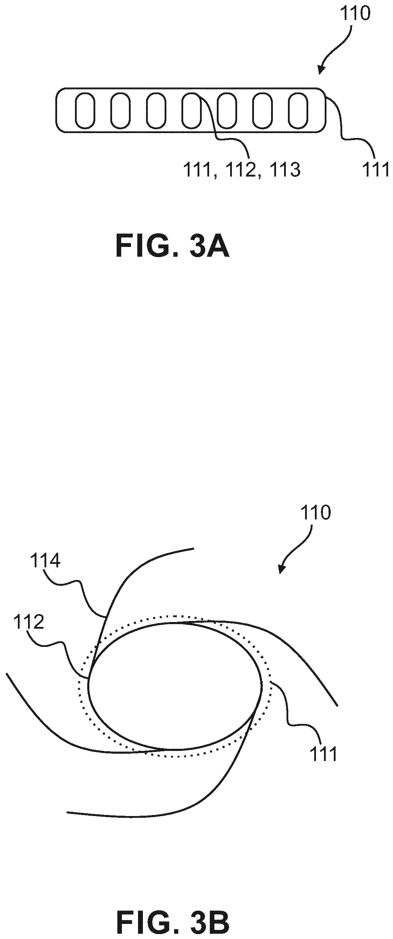

Fig. 3A to 3D show embodiments of the marker 110 to be used in the above system 100. The embodiments have in common that the marker 110 is a solid. Further, the marker 110 comprises an outer structure 111 and an inner structure 112. Preferably, the inner structure 112 comprises or forms a landmark forming component, which is configured to be detectable within the patient's P body during the medical imaging procedure to determine the movement of the patient P. This can be the detection of a mechanical body that is formed by the marker 110 or parts of it, such as the inner structure 112, or the detection of a signal that is either generated or modulated or otherwise modified by the marker 110 to form at least a kind of landmark. The outer structure 111 is configured to facilitate swallowing of the marker 110 by the patient P. In at least some embodiments, the outer structure 111 is also configured to enclose the inner structure 112 at least before and while the marker 110 is swallowed, while it can be removed after swallowing, e.g. by dissolving and/or absorbing. Further, the marker 110 or parts of it may be bio-degradable / digestible but can withstand degradation for a time interval, e.g. up to several hours, or until it enters the colon.

It is noted that the different embodiments as described herein, and particularly that below, can be combined.

Fig. 3A shows the marker 110 according to an embodiment. The outer structure 111 of this embodiment comprises or forms a, preferably flexible, housing, such as a shell etc. The outer structure 111 is configured to accommodate a set of individual markers 113. The individual markers 113 may be released within the patient's P body. Further optionally, the individual markers 113 may be assigned to different absorbing locations in the patient's P body. Optionally, the individual markers 113 may be released simultaneously, so that a clustering of markers 110 can be applied. Alternatively or additionally, the individual markers 113 are to be released in a defined way, such as a defined sequence, or the like. Optionally, the individual markers 113 may contain different concentration of a contrast agent, or the like. It is noted that one, some or all of the individual markers 113 may have the configuration as described above comprising the outer structure 111 and the inner structure 112, as indicated in Fig. 3A. Alternatively, one, some or all of the individual markers 113 do not have a multi-part structure, e.g. if they are only to be passively detected as mechanical bodies.

Fig. 3B shows the marker 110 according to an embodiment. The marker 110 comprises the outer structure 111, which is here indicated by a dashed line, and the inner structure 112. The latter comprises an actuator device 114, which may be configured to, at least temporarily, hold the marker 110 inside the patient's P body at a certain position and, optionally, for a certain time interval. This can be achieved, for example, by the actuator device 114 coming into contact with an inner wall of an organ or the like of the patient's P body, holding on, or the like. The actuator device 114 may comprise one or more of an abutment means, a friction-enhancing means, or the like. For example, the actuator device 114 may comprise one or more spring-like elements or spring elements. Optionally, these may be configured to be dissolvable and/or absorbed by the human body after a certain time interval. The outer structure 111, for example, is formed in such a way that it encloses the actuator device 114, at least while swallowing, and thus holds it together, until it removes, e.g. dissolves, for example by a suitable pH value within the patient's P body.

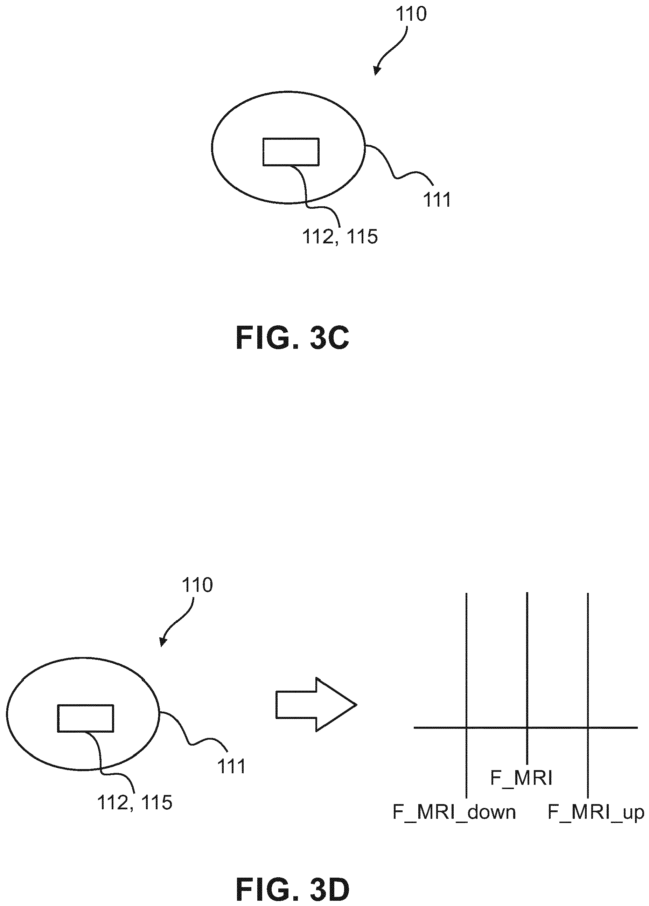

Fig. 3C shows an embodiment of the marker 110. The marker 110 comprises the outer structure 111 and the inner structure 112, and is formed as a microelectronic marker. As indicated in Fig. 3C by way of example, the inner structure 112 comprises or is formed as a micro transmitter 115 that is configured to transmit a signal. Due to the patient's P movement, e.g. its breathing motion, the signal is modulated in amplitude and/or phase, and the signal is to be detected by the medical imaging system 1000, e.g. a receiver, a receiving coil thereof etc., or a separate electronic receiver located close to the medical imaging device 1100.

Fig. 3D shows an embodiment of the marker 110, and its operating principle as a diagram. The marker 110 comprises the outer structure 111 and the inner structure 112, and is formed as a dedicated micro MRI marker. It comprises or consists of an active MRI fiducial device, which may also be referred to as the inner structure 112. As can be seen in the diagram of Fig. 3D, the fiducial device 112 is configured to electronically modulate a resonance frequency to a higher or lower frequency. Thus, a contrast can be created by B1 field enhancement or field cancellation, e.g. a destructive interference. An MRI system, i.e. the medical imaging system 1000, can detect the slight variations in intensity and phase by a separate correlator SW in k-space.

In at least some embodiments, the marker 110 has an imbalance, preferably an uneven weight distribution. This can help the marker 110 to take a preferred orientation and/or position.

In at least some embodiments, the marker 110 comprises a magnet field reacting and/or dependent component to perform a change of position and/or orientation in response to a magnetic field acting thereon. This can help to bring the marker 110 into a preferred direction with respect to e.g. a patient table, the patient body etc., when the magnetic field is a guiding parameter.

In at least some embodiments, the marker 110 has first dimensions prior to swallowing by the patient and has second dimensions within the patient P by reaction with the body, such as the pH value. Thereby, the second dimensions are greater than the first dimensions to facilitate swallowing of the marker 110.

In at least some embodiments, the landmark forming component is configured by a solid material responsive to the medical imaging technique used in the medical imaging procedure. For example, if the marker 110 or a part thereof is made from a responsive material, the material can be exploited to extend or decrease the time spent by the marker 110 in a particular part of the body. For example a marker to stay for a longer time in a specific place could be configured to, e.g. temporarily, increase its size at the desired position. Such a size change can be driven externally, as e.g. a magnetic responsive material can change shape when a magnetic field is applied and acts thereon.

In at least some embodiments, the material responsive to the medical imaging technique comprises iron. It produces a signal void in a magnitude image and the typical pattern according to its magnetic moment in a phase image. The iron content may be chosen such that the size of the signal void does not obscure anatomy. Further, several markers 110 with different iron content may be provided that can be chosen depending on e.g. the type of medical imaging, e.g. MRI, sequence to be applied. Iron doping may provide distinct patterns, as for example two dot-like artefacts at the ends of a rod-shaped tablet.

In at least some embodiments, the landmark forming component comprises a radiation absorbing material. The absorbing material content may be chosen to provide a sufficient absorption but still not beam stopping to minimize signal artefacts on 3D reconstructions. The shape of the absorbing material part may be chosen to be clearly identifiable on e.g. raw images by fully automatic image processing.

In at least some embodiments, the marker 110 has a tablet shape in which the landmark forming component is integrated.

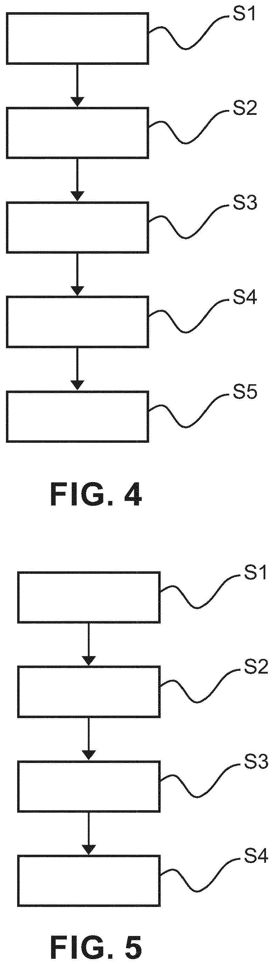

Fig. 4 shows in a flow chart the use of the one or more markers 110 as described above for determining patient movement in medical imaging.

In a step S1, the marker 110, or a plurality of identical or different markers 110, is administered to the patient P by swallowing at an estimated administration time. As described above, the administration time may be estimated and/or predicted by utilizing a model and/or simulation, or the like, based on several input parameters, such as patient information, an medical imaging information, a biomedical and/or physiological state of the patient P, and/or a scan protocol.

In a step S2, the medical imaging procedure is, preferably automatically, started at an estimated medical imaging starting time, wherein the medical imaging starting time is dependent from the administration time. As described above, the medical imaging starting time is estimated and/or predicted by utilizing a model and/or simulation, or the like, based on several input parameters. For example, the configuration of the one or more markers 110 is considered.

In a step S3, the marker 110 is detected within the patient P by using detection means. Thereby, the marker 110 forms a landmark in at least one position within the patient P. Depending on the configuration of the one or more markers 110 used, the detection means may be the medical imaging device 1100 whose images are processed to detect the marker 110 , a receiver which receives a signal sent or modified by the marker 110, or the like.

In a step S4, the data processing unit 120 determines, based on the landmark formed by the one or more markers 110 and a surrounding tissue and/or anatomy of the patient P a respirational state of the patient P during the medical imaging procedure. For example, relative movements can be detected using the one or more markers 110 or signals sent or modified by the one or more markers 110 can be received.

In a step S5, triggering or gating image acquisition during the medical imaging procedure based on the determined respirational state of the patient P is controlled. This control may be carried out by the data processing unit 120 or a control system of the medical imaging device 1100.

Fig. 5 shows in flow chart a computer-implemented method of determining patient movement for medical imaging. The method may be carried out by the above system 100 and/or the medical imaging system 1000.

In a step S1, the data processing unit 120 obtains patient information data associated with the patient P.

In the same step S1, the data processing unit 120 obtains medical imaging information data, at least indicative for a type of medical imaging procedure intended for the patient. It is noted that both of the above steps designated with 1 can also be performed successively in any order.

In a step S2, based on at least the patient information data and the medical imaging information data, the one or more markers 110 are selected and/or provided. The selection of the marker 110 may depend, for example, on the patient's P body type, the functioning of the digestive system, state of health, etc. In addition, the selection may depend on which medical imaging method is used, as the markers differ in this respect, especially with regard to the forming of the landmark.

In a step S3, the data processing unit 120 estimates and/or predicts, based on at least the patient information data and the medical imaging information data, a position and/or distribution of the one or more markers 110 inside the patient P to a certain time and/or over time, after swallowing by the patient P.

In a step S4, the data processing unit 120 generates, based on the estimation and/or prediction of the position and/or the distribution of the one or more markers 110, control data for timely controlling the medical imaging procedure. The control data may be used for triggering or gating image acquisition during the medical imaging procedure based on the determined respirational state of the patient.

The following detailed embodiments provide further details on how the at least one marker 110, the system 100 for determining patient movement for a medical imaging system 1000, and the method or determining patient movement for a medical imaging system can be realised as would be appreciated by the skilled person.

### Embodiment 1: Marker design for 3DRX and CT

Optionally, the at least one marker 110 may be designed with a core, i.e. the inner structure 112, made from a radiation absorbing material, such as iodine, calcium, or the like, and a more radiation transparent shell, i.e. the outer structure 111. The latter may have an outer shape that facilitates swallowing, and/or a surface treatment, such as a film etc., for facilitating swallowing. Optionally, a radiation absorber content may be chosen to have high absorption but still not beam stopping to minimize signal artefacts on 3D reconstructions. The shape of the radiation absorbing core may be chosen so that it easily identifiable on the raw images by fully automatic image processing, such as image analysis.

### Embodiment 2: Marker design for MR

Optionally, the at least one marker 110 may be designed as a tablet. For example, it may comprise iron content so that it can produce a signal void in a magnitude image and a typical pattern according to its magnetic moment in a phase image. The iron content may chose such that the size of the signal void does not obscure anatomy. In at least some embodiment, a plurality of markers 110 having different iron content may be provided, which markers may be chosen depending on the type of MR sequence to be applied. Iron doping may provide distinct patterns as, for example, two dot-like artefacts at the ends of a rod-shaped tablet. Optionally, the tablet may contain a material that is compact during swallowing and swells or extends once located in the digestive system of the patient, so that the size of the at least one marker 110 meets requirement of a scan resolution for fast MR imaging, which may require equal or less than 5 mm in size. or extends once digested. This allows the distinct pattern to cover larger distances and thus be identifiable at low scan resolution.

### Embodiment 3: Further marker design options

Note that the marker design options according to Embodiment 3 may be combined with the above marker design options according to Embodiments 1 and 2.

Optionally, the at least one marker 110 may comprise a, preferably flexible, housing, which may contain a set and/or a plurality of individual markers, such as 2, 3, 4, 5, 6, 7 or more individual or identical markers. The plurality of markers may be released from the housing after swallowing by, for example, dissolving the housing in response to the digestive system of the patient. Further optionally, the individual marker may be adapted to different absorbing locations in the body. Optionally, the plurality of individual markers may be released simultaneously, so that a clustering of markers may be applied. Or the markers may be released in a specific sequence. Optionally, the individual markers may contain different concentrations of a contrast agent.

Optionally, the marker 110 may comprise an actuator device 114, which may be configured to, at least temporarily, hold the marker 110 inside the body at a certain position for a certain time interval. The actuator 114 may comprise one or more of an abutment means, a friction-enhancing means, or the like. For example, the actuator device 114 may comprise one or more spring-like elements or spring elements. Optionally, these may be configured to be dissolvable and/or absorbed by the human body after a certain time interval.

Optionally, the marker 110 may be formed as a microelectronic marker. It may comprise, for example, a micro transmitter, or the like. Optionally, this marker may be configured to generate a modulated signal depending on the breathing motion detected by e.g. a sensor, receiver, or the like. For example, the signal may be modulated in amplitude and/or phase, so that the signal may be detected by an MRI system or a separate electronic receiver located close to a diagnostic imaging scanner. A CT or X-Ray scanner may comprise an extra electronic receive infrastructure, which may be located close to the X-Ray source or opposite close to the detector.

Optionally, the marker 110 may be configured as a, preferably dedicated, micro MRI marker, which may comprise an active MRI fiducial device. The fiducial device may be configured to electronically modulate a resonance frequency to a higher or lower frequency, so that a contrast may be created by a B1 field enhancement or field cancellation (destructive interference). The MRI system may detect the slight variations in intensity and phase by a separate correlator software in k-space.

It has to be noted that embodiments of the invention are described with reference to different subject matter. In particular, some embodiments are described with reference to method type claims whereas other embodiments are described with reference to device or system type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 1000: medical imaging system
- 1100: medical imaging device
- 100: system
- 110: marker
- 111: outer structure
- 112: inner structure
- 113: individual marker
- 114: actuator device
- 115: transmitter / electronic device
- 120: data processing unit
- 130: display
- S1-S5: method steps / using steps

## Claims

1. System (100) for determining patient (P) movement for a medical imaging system, comprising:
- at least one marker (110), and
- at least one data processing unit (120),
- wherein the marker (110) is a solid configured to be swallowed by the patient (P),
- wherein the marker (110) comprises a landmark forming component (111) configured to be detectable within the patient during a medical imaging procedure to determine the movement of the patient,
- wherein the at least one data processing unit (120) is configured to obtain patient information data and/or medical imaging information data at least indicative for a type of medical imaging procedure intended for the patient,
- wherein the at least one data processing unit (120) utilizes a computational model to estimate, based on one or more of the patient information data, the medical imaging information data and a configuration of the at least one marker, a position and/or distribution of the at least one marker inside the patient to a certain time and/or over a period of time after swallowing by the patient (P), and
- wherein the data processing unit (120) is configured to generate, based on the estimation of a distribution of the at least one marker, control data for timely controlling the medical imaging procedure.

2. The system according to claim 1, wherein
the control data comprises at least an administration time stamp which is derived from an estimation of an administration time at which the at least one marker is to be swallowed by the patient.

3. The system according to claim 1 or 2, wherein
the control data comprises at least a medical imaging starting time stamp which is derived from an estimation of a medical imaging starting time being dependent from an administration time at which the at least one marker is to be swallowed by the patient.

4. The system according to any one of the preceding claims, wherein
the control data comprises or feeds a medical imaging scan protocol configured to control image acquisition during the medical imaging procedure.

5. The system according to any one of the preceding claims, wherein
the at least one marker (110) has a distinct shape, preferably the shape having a spherical portion.

6. The system according to any one of the preceding claims, wherein
the at least one marker (110) has an imbalance, preferably an uneven weight distribution.

7. The system according to any one of the preceding claims, wherein
the at least one marker (110) comprises a magnet field reacting component to perform a change of position and/or orientation in response to a magnetic field acting thereon.

8. The system according to any one of the preceding claims, wherein
the at least one marker (110) has first dimensions prior to swallowing by the patient and has second dimensions within the patient (P) by reaction with the body, the second dimensions being greater than the first dimensions to facilitate swallowing of the marker (110).

9. The system according to any one of the preceding claims, wherein
the landmark forming component is configured by a solid material responsive to the medical imaging technique used in the medical imaging procedure.

10. The system according to any one of the preceding claims, wherein
the material responsive to the medical imaging technique comprises iron.

11. The system according to any one of the preceding claims, wherein
the landmark forming component comprises a radiation absorbing material.

12. The system according to any one of the preceding claims, wherein
the at least one marker (110) has a tablet shape in which the landmark forming component is integrated.

13. A marker (110) for a system (100) for determining patient movement for a medical imaging system, the marker comprising:
- an outer structure (111),
- a landmark forming component (112),
- wherein the marker (110) is a solid, the outer structure (111) being configured to facilitate swallowing by the patient (P),
- wherein the landmark forming component (112) is configured to be detectable within the patient (P) during a medical imaging procedure to determine the movement of the patient (P).

14. The use of a marker (110) according to claim 13 for determining patient movement in medical imaging, comprising:
- administrating (S1) the marker (110) to the patient by swallowing at an estimated administration time,
- starting (S2) a medical imaging procedure at an estimated medical imaging starting time, the medical imaging starting time being dependent from the administration time,
- detecting (S3), by detecting means (1100), the marker within the patient, the marker forming a landmark in at least one position within the patient,
- determining (S4), by a data processing unit (120), based on the landmark formed by the at least one marker and a surrounding anatomy of the patient a respirational state of the patient during the medical imaging procedure, and
- controlling (S5) triggering or gating image acquisition during the medical imaging procedure based on the determined respirational state of the patient.

15. A computer-implemented method of determining patient movement for medical imaging, comprising:
- obtaining (S1), by a data processing unit, patient information data,
- obtaining (S1), by the data processing unit, medical imaging information data, at least indicative for a type of medical imaging procedure intended for the patient,
- providing (S2), based on at least the patient information data and the medical imaging information data, at least one marker (110), the marker (110) being a solid configured to be swallowed by the patient, and configured to form a landmark inside the patient detectable over a period of time during the medical imaging procedure to determine the movement of the patient,
- estimating (S3), by the data processing unit (120), based on at least the patient information data and the medical imaging information data, a distribution of the marker inside the patient over time after swallowing by the patient, and
- generating (S4), by the data processing unit (120), based on the estimation of position and/or distribution of the marker (110), control data for timely controlling the medical imaging procedure.
